# FASCICULE DE BREVET EUROPEEN

(11) **EP 2 705 761 B1**
(45) Date de publication et mention de la délivrance du brevet: **11.07.2018**
(21) Numéro de dépôt: 13182819.6
(22) Date de dépôt: 03.09.2013
(51) Int. Cl.: A23L 33/105, A23L 33/15, A23L 33/16, A23L 33/10

(54) **Composition nutritionnelle pour femme enceinte**
Ernährungszusammensetzung für schwangere Frauen
Nutritional composition for pregnant women

(30) Priorité: 10.09.2012 FR 1258463
(43) Date de publication de la demande: 12.03.2014
(73) Titulaire: Laboratoires France Bebe Nutrition, 53000 Laval (FR)
(72) Inventeur: Tek, Konthirith, 53000 LAVAL (FR); Courtin, Solenn, 53000 LAVAL (FR); Guillemet, Morgane, 53000 LAVAL (FR); Giordano, Thierry, 53000 LAVAL (FR)
(74) Mandataire: Cabinet Le Guen Maillet

(56) Documents cités:
- WO-A1-00/66082
- WO-A2-03/017945
- DE-U1-202011 105 533
- FR-A1- 2 948 537
- US-A1- 2007 048 367
- US-A1- 2011 311 684

## Description

La présente invention concerne une composition nutritionnelle pour femme enceinte permettant à la fois de répondre aux besoins nutritifs liés à l'état de grossesse et de soulager le phénomène de nausée.

Durant la grossesse, les besoins nutritifs sont accrus d'une part pour le maintien de l'état physiologique de la femme et d'autre part pour le bon développement du foetus.

A titre d'exemple, les lipides et principalement les acides gras polyinsaturés sont importants pour le développement du système nerveux du bébé. Les vitamines, notamment la vitamine B9 sont essentielles à la croissance foetale. Le calcium contribue à la minéralisation osseuse. Une carence en fer contribue à la naissance prématurée.

Si l'alimentation classique permet d'apporter bon nombre de nutriments indispensables au bon déroulement de la grossesse, il convient de prendre en compte le fait que ces apports doivent couvrir les besoins de la femme et ceux du foetus. Il n'est donc pas toujours évident pour la femme de répondre à l'ensemble de ces besoins via ses habitudes alimentaires. En outre, il est difficile de déterminer si tous les apports quotidiens recommandés pour chaque nutriment sont effectivement ingérés en se basant sur l'alimentation classique.

Par ailleurs, la nausée est un symptôme courant du premier trimestre de grossesse. Elle peut parfois se manifester de manière constante tout au long de la journée et dépasser le stade du simple désagrément allant jusqu'à des vomissements intensifs. Peu de solutions sans danger pour l'état de grossesse sont aujourd'hui à la portée des femmes enceintes pour diminuer le phénomène de nausée puisque la prise de médicaments est fortement déconseillée. En outre, l'état de nausée aboutit à une sous-alimentation de la femme enceinte, et ainsi à des carences en éléments nutritifs essentiels. Les documents US 2011/0311684 A1 et WO 03/017945 A2 décrivent l'effet de la vitamine B6 et du gingembre pour réduire la nausée chez la femme enceinte. Le but de la présente invention est ainsi de proposer une composition nutritionnelle pour femmes enceintes qui permet, d'une part, de couvrir les besoins nutritionnels spécifiques liés à l'état de grossesse et d'autre part, de réduire le phénomène de nausée.

A cet effet, l'invention concerne une composition nutritive comprenant une fraction protéique, une fraction glucidique, une fraction lipidique comprenant au moins un acide gras du groupe des omégas 3, au moins un élément minéral dont le fer, au moins une vitamine dont la vitamine B6 et la combinaison d'une poudre de gingembre et d'un extrait d'artichaut.

Les plantes sont aujourd'hui très souvent associées à l'alimentation humaine ou animale en raison de leurs nombreuses vertus. Elles présentent en effet des propriétés physiologiques intéressantes chez l'homme, ou l'animal et leur exploitation dans des compositions nutritives, des compléments alimentaires ou des compositions pharmaceutiques est aujourd'hui bien accueillie.

L'extrait d'artichaut, *Cynara scolymus*, est obtenu par un procédé conventionnel d'extraction à partir des parties aériennes de la plante avec de l'eau ou un mélange eau/alcool en tant que solvant. De tels procédés sont décrits dans la littérature. De plus, de tels extraits d'artichauts sont trouvés couramment dans le commerce et ceux-ci conviennent dans le cadre de l'invention.

Le gingembre, *Zingiber officinale*, est préférentiellement sous forme d'une poudre obtenue par séchage puis broyage du rhizome.

Le gingembre présente une activité antiémétique et l'artichaut des propriétés antidyspeptique ou cholérétique.

La composition selon l'invention contient ainsi des protéines, des lipides, des glucides ainsi que des vitamines et des minéraux qui sont nécessaires aux besoins de la femme enceinte et à l'enfant qu'elle porte. Tous ces éléments nutritifs sont réunis dans une même composition ce qui évite de multiplier les prises. Elle présente l'avantage supplémentaire de contenir un mélange d'extraits de plantes permettant de diminuer le phénomène de nausée. Cette composition est ainsi parfaitement adaptée à la condition de la femme durant la grossesse.

La fraction protéique peut être d'origine variée. Il peut s'agir de protéines d'origine animale, végétale, laitière ou un mélange. La source de protéines peut être, par exemple, constituée par un lait entier, demi-écrémé ou écrémé. Des céréales peuvent également composer la source de protéines.

La fraction lipidique peut être obtenue à partir d'huile ou de graisse végétale ou animale, laitière, à partir d'oeufs ou encore à partir d'huile de poisson ou d'algues. De même, toute source de glucides peut être envisagée.

La composition selon l'invention inclut dans sa fraction lipidique au moins un acide gras dit du groupe des omégas 3. Les acides gras du groupe des omégas 3 sont par exemple l'acide eicosapentaénoïque (EPA), l'acide docosahexaénoïque (DHA), l'acide alpha linolénique (ALA).

Selon un mode préféré de réalisation de l'invention, l'acide gras du groupe des omégas 3 dans la fraction lipidique est l'acide alpha linolénique (ALA) ou l'acide docosahexaénoïque (DHA).

La vitamine B6 contribue également à diminuer le phénomène de nausée. Toutefois, la composition peut comprendre, de plus, une ou plusieurs autres vitamines telles que la vitamine A, la vitamine B1, la vitamine B2, la vitamine B3, la vitamine D, la vitamine B9, la vitamine B12, la vitamine E, la vitamine C. Préférentiellement, elle comprend de la vitamine B9 et/ou de la vitamine D, en plus de la vitamine B6. Préférentiellement encore, la composition comprend un mélange de vitamines B6, B9 et D. La vitamine B9 est connue pour favoriser le développement du tube neural et il est aujourd'hui vivement conseillé aux femmes enceintes d'en absorber quotidiennement.

La composition selon l'invention peut comprendre d'autres minéraux en plus du fer. Ainsi, selon un mode de réalisation de l'invention, la composition comprend, en plus du fer, de l'iode et/ou du magnésium. Préférentiellement encore, la composition comprend un mélange de fer, d'iode et de magnésium.

L'iode est présent dans la composition principalement sous forme d'iodure de potassium ou de toute autre forme autorisée.

Le fer est apporté sous forme de lactate ou de toute autre forme autorisée.

Le magnésium est apporté sous forme de carbonate de magnésium ou de toute autre forme autorisée.

Selon un mode de réalisation de l'invention, la composition se présente sous forme d'un aliment dont le taux d'humidité est inférieur à 10%, préférentiellement sous forme d'un biscuit ou d'une barre céréalière.

Avantageusement, la composition comprend une base céréalière, préférentiellement choisie parmi le blé, le seigle, l'orge, le maïs, le riz, l'avoine, le sorgho, le millet, le sarrasin, le quinoa et l'épeautre. De plus, une composition formulée sous forme de biscuit présente l'avantage d'être plus facile à ingérer que des comprimés.

L'invention concerne encore une telle composition sous la forme d'une dose formée de :
- entre 0,5 et 3 mg, en particulier entre 1 et 2 mg de vitamine B6,
- entre 10 et 200 µg, en particulier entre 50 et 150 µg de vitamine B9,
- entre 1 et 5 µg, en particulier entre 1,5 et 3 µg de vitamine D,
- entre 10 et 200 µg, en particulier entre 40 et 100 µg d'iode,
- entre 50 et 200 mg de magnésium,
- entre 5 et 15 mg de fer,
- entre 0,01 et 1,5 g d'acides gras polyinsaturés à longue chaîne du groupe des omégas 3,
- entre 200 et 2000 mg, en particulier entre 500 et 1500 mg, d'un extrait artichaut tel que décrit précédemment,
- entre 500 et 2000 mg, en particulier entre 1000 et 2000 mg, de poudre de rhizome de gingembre.

La dose est préférentiellement quotidienne, c'est-à-dire que la composition apporte quotidiennement les quantités précitées.

L'apport quotidien pourra par exemple être réalisé par le biais d'une formulation unique de 80 g ou réparti en différentes sous-doses sur une journée.

Selon un autre mode de réalisation, la composition se présente sous la forme d'un liquide, d'une poudre, d'un aliment solide ou semi-solide, d'un comprimé, d'un granulé, d'une solution contenue dans une ampoule ou de toute autre forme galénique appropriée.

L'invention concerne l'utilisation d'une composition ou d'une dose d'une composition telle que décrite précédemment dans l'alimentation de la femme enceinte, en particulier pour combler les besoins en éléments nutritifs liés à l'état de grossesse. Ces éléments nutritifs sont ceux décrits précédemment.

L'invention peut bien entendue être appliquée à tout autre mammifère.

La composition est ingérée quotidiennement, préférentiellement fractionnée en sous-doses.

L'invention est illustrée dans les exemples qui suivent qui se veulent illustratifs et non limitatifs.

### Composition nutritive pour femme enceinte se présentant sous forme d'un biscuit prêt à consommer :

### Quantité/100 g

Vitamine B6 : 2 mg
Vitamine B9 : 125 µg
Vitamine D : 3 µg
Iode : 63 µg
Magnésium : 141 µg
Fer : 9 mg
Lipides : 20 g
Protéines : 8 g
Glucides : 65 g
dont sucres: 20 g
Extrait d'artichaut : 1 g
Poudre de gingembre : 1.5 g

## Revendications

1. Composition nutritive comprenant :
- une fraction protéique,
- une fraction glucidique,
- une fraction lipidique comprenant au moins un acide gras du groupe des omégas 3,
- au moins un élément minéral dont le fer,
- au moins une vitamine dont la vitamine B6,
- la combinaison d'une poudre de gingembre et d'un extrait des parties aériennes d'artichaut, lequel extrait étant obtenu par extraction avec de l'eau ou un mélange eau/alcool.

2. Composition selon la revendication 1, **caractérisée en ce qu'**elle comprend une base céréalière préférentiellement choisie parmi le blé, le seigle, l'orge, le maïs, le riz, l'avoine, le sorgho, le millet, le sarrasin, le quinoa et/ou l'épeautre.

3. Composition selon la revendication 1, **caractérisée en ce qu'**elle se présente sous la forme d'un aliment dont le taux d'humidité est inférieur à 10%, préférentiellement sous la forme d'un biscuit ou d'une barre de céréales.

4. Composition selon l'une des revendications 1 à 3, **caractérisée en ce que** ledit acide gras du groupe des omégas 3 est l'acide alphalinolénique (ALA) ou l'acide docosahexaénoïque (DHA).

5. Composition selon l'une des revendications 1 à 4, **caractérisée en ce qu'**elle comprend, de plus, parmi les minéraux, de l'iode et/ou du magnésium.

6. Composition selon l'une des revendications 1 à 5, **caractérisée en ce qu'**elle comprend, de plus, parmi les vitamines, de la vitamine B9 et/ou de la vitamine D.

7. Composition selon l'une des revendications 1 à 6, **caractérisée en ce qu'**elle se présente sous la forme d'un liquide, d'une poudre, d'un aliment, d'un comprimé, d'un granulé, d'une solution contenue dans une ampoule ou de toute autre forme galénique appropriée.

8. Composition selon l'une des revendications 1 à 7, **caractérisée en ce qu'**elle se présente sous la forme d'une dose formée de :
- entre 0,5 et 3 mg, en particulier entre 1 et 2 mg de vitamine B6,
- entre 10 et 200 µg, en particulier entre 50 et 150 µg de vitamine B9,
- entre 1 et 5 µg, en particulier entre 1,5 et 3 µg de vitamine D,
- entre 10 et 200 µg, en particulier entre 40 et 100 µg d'iode,
- entre 50 et 200 mg de magnésium,
- entre 5 et 15 mg de fer,
- entre 0,01 et 1,5 g d'acides gras polyinsaturés à longue chaîne du groupe des omégas 3,
- entre 200 et 2000 mg, en particulier entre 500 et 1500 mg, d'un extrait d'artichaut, lequel extrait étant obtenu par extraction avec de l'eau ou un mélange eau/alcool,
- entre 500 et 2000 mg, en particulier entre 1000 et 2000 mg, de poudre de rhizome de gingembre.

9. Utilisation d'une composition telle que définie dans l'une des revendications 1 à 8 dans l'alimentation de la femme enceinte, en particulier pour combler les besoins en éléments nutritifs.

## Patentansprüche

1. Nahrungsmittelzusammensetzung, umfassend:
- eine Proteinfraktion,
- eine Kohlenhydratfraktion,
- eine Lipidfraktion, die mindestens eine Fettsäure der Omega-3-Gruppe umfasst,
- mindestens ein Mineralienelement, einschließlich Eisen,
- mindestens ein Vitamin, einschließlich Vitamin B6,
- die Kombination aus einem Ingwerpulver und einem Extrakt oberirdischer Teile der Artischocke, wobei der Extrakt durch Extraktion mit Wasser oder einem Wasser/Alkohol-Gemisch erhalten wird.

2. Zusammensetzung nach Anspruch 1, **dadurch gekennzeichnet, dass** sie eine Zerealiengrundlage, die vorzugsweise aus Weizen, Roggen, Gerste, Mais, Reis, Hafer, Sorghum, Hirse, Buchweizen, Quinoa und/oder Dinkel ausgewählt ist, umfasst.

3. Zusammensetzung nach Anspruch 1, **dadurch gekennzeichnet, dass** sie in Form eines Nahrungsmittels, dessen Feuchtigkeitsgehalt niedriger als 10% ist, vorzugsweise in Form eines Kekses oder eines Zerealienriegels bereitgestellt wird.

4. Zusammensetzung nach einem der Ansprüche 1 bis 3, **dadurch gekennzeichnet, dass** die besagte Fettsäure der Omega-3-Gruppe alpha-Linolensäure (ALA) oder Docosahexaensäure (DHA) ist.

5. Zusammensetzung nach einem der Ansprüche 1 bis 4, **dadurch gekennzeichnet, dass** sie weiterhin, unter den Mineralien, Iod und/oder Magnesium umfasst.

6. Zusammensetzung nach einem der Ansprüche 1 bis 5, **dadurch gekennzeichnet, dass** sie weiterhin, unter den Vitaminen, Vitamin B9 und/oder Vitamin D umfasst.

7. Zusammensetzung nach einem der Ansprüche 1 bis 6, **dadurch gekennzeichnet, dass** sie in Form einer Flüssigkeit, eines Pulvers, eines Nahrungsmittels, einer Tablette, eines Granulats, einer Lösung, die in einer Ampulle enthalten ist, oder in irgendeiner anderen geeigneten Darreichungsform bereitgestellt wird.

8. Zusammensetzung nach einem der Ansprüche 1 bis 7, **dadurch gekennzeichnet, dass** sie in einer Dosierungsform bereitgestellt wird, die Folgendes umfasst:
- zwischen 0,5 und 3 mg, insbesondere zwischen 1 und 2 mg Vitamin B6,
- zwischen 10 und 200 µg, insbesondere zwischen 50 und 150 µg Vitamin B9,
- zwischen 1 und 5 µg, insbesondere zwischen 1,5 und 3 µg Vitamin D,
- zwischen 10 und 200 µg, insbesondere zwischen 40 und 100 µg Iod,
- zwischen 50 und 200 mg Magnesium,
- zwischen 5 und 15 mg Eisen,
- zwischen 0,01 und 1,5 g langkettige polyungesättigte Fettsäuren der Omega-3-Gruppe,
- zwischen 200 und 2000 mg, insbesondere zwischen 500 und 1500 mg eines Extraktes aus Artischocke, wobei der Extrakt durch Extraktion mit Wasser oder einem Wasser/Alkohol-Gemisch erhalten wird,
- zwischen 500 und 2000 mg, insbesondere zwischen 1000 und 2000 mg Ingwerwurzelpulver.

9. Verwendung einer Zusammensetzung, wie in einem der Ansprüche 1 bis 8 definiert, in der Ernährung von schwangeren Frauen, insbesondere um den Bedarf an Nährstoffelementen zu erfüllen.

## Claims

1. Nutritional composition comprising:
- a protein fraction,
- a carbohydrate fraction,
- a lipid fraction comprising at least one fatty acid from the omega-3 group,
- at least one mineral element, including iron,
- at least one vitamin, including vitamin B6,
- a combination of powdered ginger and an extract of the shoot system of the artichoke, said extract being obtained by extraction with water or with a water/alcohol mixture.

2. Composition according to claim 1, **characterised in that** it comprises a cereal base preferably selected from wheat, rye, barley, maize, rice, oats, sorghum, millet, buckwheat, quinoa and/or spelt.

3. Composition according to claim 1, **characterised in that** takes the form of a foodstuff whose moisture content is less than 10%, and preferably the form of a biscuit or cereal bar.

4. Composition according to one of claims 1 to 3, **characterised in that** said fatty acid from the omega-3 group is alpha-linolenic acid (ALA) or docosahexaenoic acid (DHA).

5. Composition according to one of claims 1 to 4, **characterised in that**, of the minerals, it comprises in addition iodine and/or magnesium.

6. Composition according to one of claims 1 to 5, **characterised in that**, of the vitamins, it comprises in addition vitamin B9 and/or vitamin D.

7. Composition according to one of claims 1 to 6, **characterised in that** it takes the form of a liquid, a powder, a foodstuff, a tablet, a granular material, a solution contained in an ampoule, or any other suitable dosage form.

8. Composition according to one of claims 1 to 7, **characterised in that** it takes the form of a dose formed by:
- between 0.5 and 3 mg, and in particular between 1 and 2 mg, of vitamin B6,
- between 10 and 200 µg, and in particular between 50 and 150 µg, of vitamin B9,
- between 1 and 5 µg, and in particular between 1.5 and 3 µg, of vitamin D,
- between 10 and 200 µg, and in particular between 40 and 100 µg, of iodine,
- between 50 and 200 mg of magnesium,
- between 5 and 15 mg of iron,
- between 0.01 and 1.5 g of long-chain polyunsaturated fatty acids from the omega-3 group,
- between 200 and 2000 mg, and in particular between 500 and 1500 mg, of an artichoke extract, said extract being obtained by extraction with water or with a water/alcohol mixture,
- between 500 and 2000 mg, and in particular between 1000 and 2000 mg, of powdered ginger rhizome.

9. Use of a composition as defined in one of claims 1 to 8 in feeding pregnant women, in particular to meet their nutritional requirements.
